# EUROPÄISCHE PATENTSCHRIFT

(11) **EP 0 973 480 B1**
(45) Veröffentlichungstag und Bekanntmachung des Hinweises auf die Patenterteilung: **10.09.2003**
(21) Anmeldenummer: 98912496.1
(22) Anmeldetag: 23.03.1998
(51) Int. Cl.: A61K 6/00

(54) **SILBER-PALLADIUM-LEGIERUNGEN ZUR HERSTELLUNG VON MIT DENTALKERAMIK VERBLENDBAREM ZAHNERSATZ**
SILVER-PALLADIUM ALLOYS FOR PRODUCING A DENTAL PROSTHESIS WHICH CAN BE COVERED WITH DENTAL CERAMIC
ALLIAGES ARGENT-PALLADIUM UTILISES POUR PRODUIRE UNE PROTHESE DENTAIRE POUVANT ETRE REVETUE DE CERAMIQUE DENTAIRE

(30) Priorität: 04.04.1997 DE 19713925
(43) Veröffentlichungstag der Anmeldung: 26.01.2000
(73) Patentinhaber: DeguDent GmbH, 63457 Hanau (DE)
(72) Erfinder: GRAU, Franz, Josef, D-91077 Neunkirchen am Brand (DE); VAN DER ZEL, Joseph, Maria, NL-1628 TN Hoorn (NL)
(74) Vertreter: Stoffregen, Hans-Herbert, Dr. Dipl.-Phys.
(86) Internationale Anmeldenummer: EP9801682
(87) Internationale Veröffentlichungsnummer: WO98044894

(56) Entgegenhaltungen:
- EP-A- 0 330 863
- DE-C- 3 438 288
- DE-C- 3 905 987
- NL-A- 9 200 566
- US-A- 5 290 371

## Beschreibung

Die Erfindung betrifft Silber-Palladium-Legierungen zur Herstellung von mit Dentalkeramik verblendbarem Zahnersatz.

Festsitzender und herausnehmbarer Zahnersatz wird vorwiegend aus korrosionsbeständigen, biokompatiblen Edelmetall 3 legierungen mit dem sogenannten Wachsausschmelzverfahren hergestellt, wobei das gegossene Objekt häufig mit Dentalkeramik verblendet wird, um ein dem natürlichen Zahn entsprechendes Aussehen zu erzielen. Hierfür müssen die Legierungen spezielle, auf die Dentalkeramik abgestimmte Eigenschaften besitzen, wie thermischer Ausdehnungskoeffizent, Schmelzintervall und Haftung.

Hochgoldhaltige Legierungen, wie sie z.B. in den Patentschriften DE-11 83 247 und DE-15 33 233 beschrieben sind, eignen sich für diese Zwecke besonders gut. Wegen des hohen, stark schwankenden Preises von Gold ist jedoch in neuerer Zeit verstärkt nach preiswerteren Alternativen zu den hochgoldhaltigen Legierungen gesucht worden. Im Bereich der Edelmetalle bietet sich als Einsatz das Palladium wegen seines relativ günstigen Preises, seiner gegenüber Gold deutlich verringerten Dichte und'seiner dem Gold vergleichbaren Korrosions- bzw. Mundbeständigkeit an.

Im Vergleich zu hochgoldhaltigen Legierungen weisen Palladiumbasislegierungen neben einem niedrigeren Preis eine höhere Härte und Festigkeit, eine durch das Palladium bewirkte höhere Solidustemperatur, eine bessere Hochtemperaturfestigkeit und dadurch bei Aufbrennlegierungen eine höhere Verzugsfestigkeit beim Keramikbrand auf. Diese Legierungen sind entweder goldfrei oder enthalten nur wenige Gew.% Gold. Sie reagieren jedoch empfindlicher auf Verarbeitungsfehler und sind schwer lötbar. Der Gehalt an Palladium führt zu einer Reduktion des Wärmeausdehnungskoeffizenten; dies kann allerdings durch die Zugabe von Silber ausgeglichen werden.

Silberhaltige Palladiumbasislegierungen sind bezüglich ihres Verarbeitungsverhaltens zwischen den hochgoldhaltigen und den silberfreien Palladiumbasislegierungen anzusiedeln. Im Vergleich zu silberfreien Palladiumbasislegierungen sind silberhaltige Palladiumbasislegierungen, bedingt durch den Silberanteil besser schmelz- und gießbar, besitzen ein helleres Oxid, zeigen ein gutes Lötverhalten und sind außerdem noch preiswerter.

Seit einigen Jahren ist ein Dentalsystem im Markt eingeführt, das aus einer hochgoldhaltigen Legierung, einer niedrigschmelzenden Dentalkeramik sowie den entsprechenden Lotlegierungen besteht. Im Vergleich zu bis zum damaligen Zeitpunkt verwendeten Keramiken weist die niedrigschmelzende Keramik einen wesentlich höheren Wärmeausdehnungskoeffizenten auf.

Zunehmend wird gewünscht, diese niedrigschmelzende Dentalkeramik mit einer aufbrennfähigen, preiswerteren Palladium-Silber- bzw. Silber-Palladium-Legierung zu kombinieren.

Für aufbrennfähige Palladiumbasislegierungen bedeutet jedoch ein höherer Wärmeausdehnungskoeffizient der Keramik eine deutliche Steigerung des Silbergehaltes auf ca. 50 Gew% und mehr.

Der Nachteil dieser Silberbasislegierungen bzw. Palladiumbasislegierungen mit hohem Silbergehalt ist, daß sie die Verblendkeramik während des Aufbrennprozesses gelb bzw. gelbgrün verfärben. Die Ursache hierfür ist das Silber, das durch Diffusion bzw. über die Dampfphase in die Keramik gelangt.

Die Problematik bezüglich der Verfärbung von Keramik durch Silber ist bekannt.

Für Palladium-Silberlegierungen, aufbrennfähig für konventionelle hochschmelzende Keramik mit Wärmeausdehnungskoeffizenten im Bereich von 14 µm/mK bis 15 µm/mK, wurden bis zu einem Silbergehalt von 45 Gew.% Silber Lösungen gefunden (DE3905987). Zum Aufbrennen von niedrigschmelzender Keramik mit Wärmeausdehnungskoeffizienten von ca. 16,5 µm/mK sind diese Legierungen jedoch nicht geeignet. Der Silbergehalt in solchen mit niedrigschmelzender Keramik kompatiblen Aufbrennlegierungen muß angehoben werden; der höhere Silbergehalt führt jedoch in der Regel zu einer höheren Verfärbungsneigung der Dentalkeramik durch Silber, welche auch durch die in DE-39 05 987 genannten Lösungen nicht mehr unterdrückt werden kann.

In DE-PS 25 23 971 werden Palladium-Silber-Legierungen beschrieben, die zu Unterdrückung der Keramikverfärbung 0,1 bis 0,5 % Titan enthalten. Aufgrund der Reaktivität von Titan mit dem Luftsauerstoff bzw. dem Tiegelwerkstoff verarmt die Schmelze relativ schnell an diesem Element, so daß die reduzierende Wirkung auf die Verfärbungsneigung bei Verwendung von Altmaterial (Angußkanäle, Gußtrichter) und bei ungünstig gewählten Schmelzbedingungen verloren geht. Das Titan verursacht außerdem eine starke Haftung der Einbettmasse an der Oberfläche des gegossenen Objektes, was das Ausbetten und Ausarbeiten schwieriger und zeitaufwendiger macht.

In US-Patent 4 350 526 werden Palladium-Silber-Legierungen beschrieben, die aufgrund einer Zugabe von 0,1 bis 1,0 % Silizium keine verfärbende Wirkung auf Dentalkeramik haben. Silizium ist sowohl in Palladium als auch in Silber unlöslich. Palladium und Silizium bilden außerdem intermetallische Phasen, so daß eine starke Versprödung der Legierung und Gußbrüchigkeit auftreten können.

Silizium begünstigt - ähnlich wie Titan - die Reaktion mit keramischen Werkstoffen, so daß auch bei diesen Legierungen eine starke Haftung der Einbettmasse an dem Gußobjekt auftritt.

Gemäß der DE-PS 29 42 373 sollen Zusätze von 0,01 bis 5 % Silizium, Bor und/oder Germanium bei Dentallegierungen mit 30 bis 50 % Goldgehalten die Silberverfärbung unterdrücken. Bei goldfreien oder goldarmen Palladiumlegierungen vermag allerdings Germanium allein keine solchen Effekte hervorzurufen.

In NL 9200566 werden aufbrennfähige Palladium-Silber-Dentallegierungen beschrieben, die neben weiteren Legierungsbestandteilen auch Indium, Zinn und Zink enthalten können. Diese Legierungen sind für die Verblendung mit konventioneller hochschmelzender Keramik mit einem Wärmeausdehnungskoeffizient um 14,5 µm/mK, vorgesehen. Im praktischen Einsatz hat sich jedoch herausgestellt, daß diese Legierungen starke Verfärbungen an Dentalkeramik, insbesondere des neueren niedrigschmelzenden Typs mit Wärmeausdehnungskoeffizient um 16,5 µm/mK, bewirkt.

Es war daher Aufgabe der vorliegenden Erfindung, Silber-Palladium-Legierungen zur Herstellung von festsitzendem und herausnehmbarem, mit niedrigschmelzender Dentalkeramik mit Wärmeausdehnungskoeffizienten von ca. 16,5 µm/mK verblendbarem Zahnersatz zu entwickeln, auf die derartige verfärbungsempfindliche Dentalkeramiken ohne erkennbare Farbänderungen aufgebrannt und die leicht aus den üblichen Einbettmassen ausgebettet werden können, ohne daß sich die übrigen Eigenschaften der Silber-Palladium-Legierungen signifikant ändern.

Es wurde nun gefunden, daß diese Bedingungen von Silber-Palladium-Legierungen erfüllt werden, die aus 45-65 Gew.% Ag, 30-45 Gew.% Pd, 0-5 Gew.% Au, 0-5 Gew.% Pt, 0-3 Gew.% Ge, 0-3 Gew.% Cu, 0-7 Gew.% Ga, 0-5 Gew.% Co, 0-1 Gew.% Mo, 0-1 Gew.% Ru, 0-1 Gew.% Re, 0-1 Gew.% Ir und je 0 bis 6 Gew.% In, Sn und Zn bestehen, wobei sich alle Bestandteile zu 100 % ergänzen, und die dadurch gekennzeichnet sind, daß entweder bei einem Gehalt von 0-1 Gew.% In gleichzeitig der Gehalt an Sn 1-6 Gew.% und der Gehalt an Zn 2-6 Gew.% oder bei einem Gehalt von 3-6 Gew.% In gleichzeitig der Gehalt an Sn 0-4 Gew.% und der Gehalt an Zn 4-6 Gew.% beträgt.

Gegenstand der Erfindung ist daher die Verwendung von wie vorstehend charakterisierten Silber-Palladium-Legierungen zur Herstellung von mit niedrigschmelzender Dentalkeramik mit einem Wärmeausdehnungskoeffizient von ca. 16,5 µm/mK verblendbarem Zahnersatz.

Gegenstand der Erfindung ist weiterhin die Verwendung von wie vorstehend charakterisierten Silber-Palladium-Legierungen und niedrigschmelzender Dentalkeramik mit einem Wärmeausdehnungskoeffizient von ca. 16,5 µm/mK zur Herstellung von mit Dentalkeramik verblendetem, verfärbungsfreien Zahnersatz.

Gegenstand der Erfindung ist darüberhinaus festsitzender und herausnehmbarer Zahnersatz, bei dem eine Substruktur aus einer wie vorstehend charakterisierten Silber-Palladium-Legierung mit einer niedrigschmelzenden Dentalkeramik mit Wärmeausdehnungskoeffizient von ca. 16,5 µm/mK verblendet ist.

Die erfindungsgemäßen Legierungen besitzen einen Wärmeausdehnungskoeffizienten, der zwischen 16,4 und 17,1 µm/mK liegt. Sie sind deshalb in besonderem Maße kompatibel mit niedrigschmelzender Dentalkeramik mit Wärmeausdehnungskoeffizient von ca. 16,5 µm/mK.

Überraschenderweise hat sich gezeigt, daß bezüglich der Legierungszusammensetzung Bereiche existieren, bei denen es zu keiner Verfärbung der Dentalkeramik bzw. zu einer deutlichen Reduzierung der Gelb-/Grünverfärbung von Dentalkeramiken gegenüber herkömmlichen Silber-Palladium-Legierungen kommt.

Dies trifft in den beiden Fällen zu, wenn entweder bei einem Gehalt von 0-1 Gew.% In gleichzeitig der Gehalt an Sn 1-6 Gew.% und der Gehalt an Zn 2-6 Gew.% oder bei einem Gehalt von 3-6 Gew.% In gleichzeitig der Gehalt an Sn 0-4 Gew.% und der Gehalt an Zn 4-6 Gew.% beträgt.

Neben Silber und Palladium enthalten diese Legierungen vorwiegend die Elemente Indium, Zinn und Zink in den angegebenen Anteilen. Optional können die erfindungsgemäßen Legierungen in den angegebenen Anteilen noch die Elemente Gold, Platin, Germanium, Gallium, Indium, Kobalt, Kupfer und Molybdän enthalten. Die Elemente dienen der Abstimmung der mechanischen Eigenschaften wie z.B. Festigkeit, Härte, Gießbarkeit und Schmelzintervall. Ruthenium, Rhenium und/oder Iridium können in den angegebenen Konzentrationsbereichen als Kornfeinungszusätze zulegiert sein.

Die erfindungsgemäßen Legierungen sind biokompatibel. Auf die Verwendung von toxischen und allergieauslösenden Elementen, wie zum Beispiel Cadmium oder Nickel, konnte verzichtet werden.

Einen entscheidenen Einfluß auf die Verfärbungsneigung zeigte der Gehalt an Indium in Kombination mit dem Zinkund Zinn-Gehalt. Intensive grün/gelbe Verfärbungen waren bei Indiumgehalten im Bereich um 2 Gew.% feststellbar. Bei einem niedrigen Gehalt an Indium bis max. 1 Gew.% und gleichzeitig Zinngehalten von 1-6 Gew.% war ein Minimum bezüglich Verfärbungsneigung feststellbar, wenn der Zinkgehalt bei 2-6 Gew.% lag. Verfärbungsfrei zeigten sich Dentallegierungen bei Indiumgehalten kleiner 1 Gew.% und Zinn-und Zink-Gehalten von 3 bis 5 Gew.%. In diesem Zusammensetzungsbereich wird das Silber auch in oberflächennahen Bereichen so fest durch die anderen Legierungselemente gebunden, daß eine Verfärbung durch Diffusion des Silbers bzw. eine Verfärbung der Dentalkeramik über mögliches Silber aus der Dampfphase verhindert wird.

Erfindungsgemäße Legierungen, mit niedrigem Indiumgehalt enthalten vorzugweise 50-60 Gew.% Ag, 32-45 Gew.% Pd, 0-1 Gew.% In, 3-5 Gew.% Sn und 3-5 Gew.% Zn.

Besonders bevorzugt sind indiumfreie Legierungen, die 55-57 Gew.% Ag, 35-38 Gew.% Pd, 3-5 Gew.% Sn und 3-5 Gew.% Zn enthalten.

Überraschenderweise zeigte sich bei den Untersuchungen, daß noch ein 2. Bereich bei Indiumgehalten von 3-6 Gew.% existiert, wenn der Zinkgehalt 4-6 Gew.% und der Zinngehalt 0-4 Gew.% beträgt. In diesem Zusammensetzungsbereich wird eine Barriere gegen Silberdiffusion gebildet. Wichtig ist jedoch dabei, daß der Gehalt an Indium 3 Gew.% nicht unterschreitet und der Zinngehalt nicht über 4 Gew.% liegt, da andernfalls die Silber - Diffusionsbarriere geschwächt und eine Zunahme der Verfärbungsneigung festzustellen ist. Günstiger sind diesbezüglich Legierungen mit Zinngehalten von maximal 2 Gew.% und einem Indiumgehalt von mindestens 3 Gew.%, wenn die Legierung gleichzeitig mindestens 4 Gew.% Zink enthält.

Bevorzugt sind entsprechende Legierungen die 50-60 Gew.% Ag, 32-45 Gew.% Pd, 3-6 Gew.% In, 0-2 Gew.% Sn und 4-6 Gew.% Zn enthalten.

Besonders bevorzugt sind solche Legierungen, die 50-55 Gew.% Ag, 38-42 Gew.% Pd, 3-5 Gew.% In, 0,5-2 Gew.% Sn und 4-5 Gew.% Zn enthalten.

Die erfindungsgemäß zu verwendenden Silber-Palladium-Legierungen lassen sich in bekannter Weise mit hierfür üblichen Techniken und Hilfsmitteln zu mit Dentalkeramik verblendetem, festsitzenden oder herausnehmbaren Zahnersatz verarbeiten. Dabei werden sie etwa im Wachsausschmelzverfahren zu einer Substruktur vergossen, die dann durch Aufschmelzen von niedrigschmelzender Dentalkeramik verblendet wird.

Tabelle 1 zeigt die Zusammensetzung und die Eigenschaften einiger erfindungsgemäßen Legierungen. Sie zeichnen sich durch eine hervorragende Vergießbarkeit und eine problemlose Verblendbarkeit mit bekannten, niedrigschmelzenden Dentalkeramiken mit Wärmeausdehnungskoeffizient von ca. 16,5 µm/mK aus.

Die Bestimmung der Farbe der Verblendkeramik auf den Legierungen nach dem Aufbrennen erfolgte durch eine Reflexions-Messung mittels Spektrophotometer im Vergleich zu einer Standardprobe (Palliag NF IV). Bei der Reflexions-Messung wird die Probe diffus beleuchtet und unter einen Beobachtungswinkel von 2 Grad betrachtet. Die Ergebnisse sind in Tabelle 2 zusammengestellt (CIEL System, Lichtart D 65). Der a-Wert beschreibt die Lage auf der Grün/Rot-Achse (negativ = grün, positiv = rot), der b-Wert die Lage auf der Blau-/Gelb - Achse (blau negativ, gelb positiv).Tabelle 2 enthält nicht die absoluten Farbwerte, sondern die Abweichungen der Farbwerte für die Keramikverblendung auf den verschiedenen Legierungen von der Standardprobe (Δ-Werte). Gegenüber der Standardlegierung war bei den erfindungsgemäßen Legierungen eine deutliche Reduzierung der Gelb-/Grünverfärbung meßbar. Bei visueller Betrachtung von aus erfindungsgemäßen Legierungen gefertigten und verblendeten Kronen und Brücken konnte keine Verfärbung der Keramik beobachtet werden.

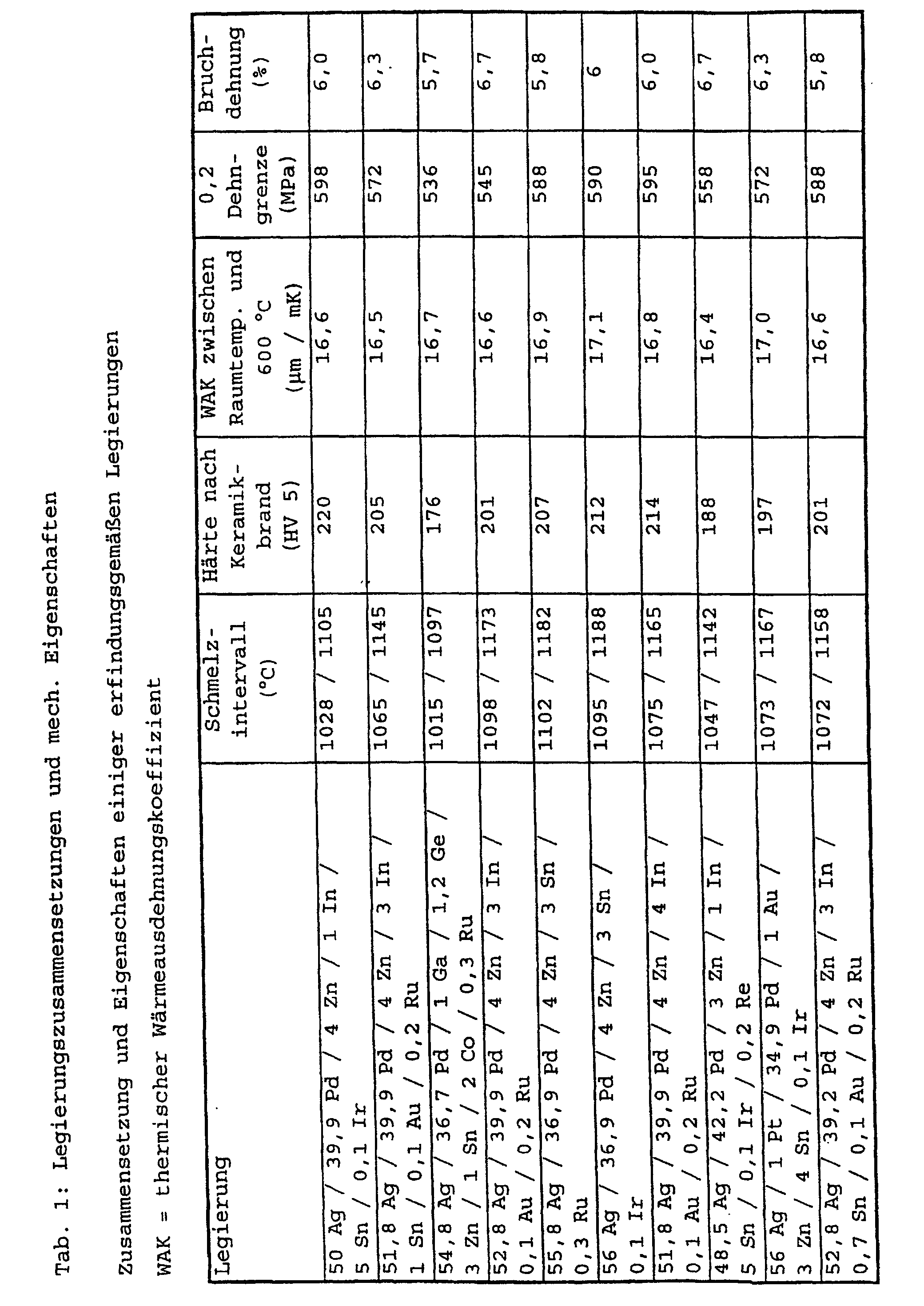

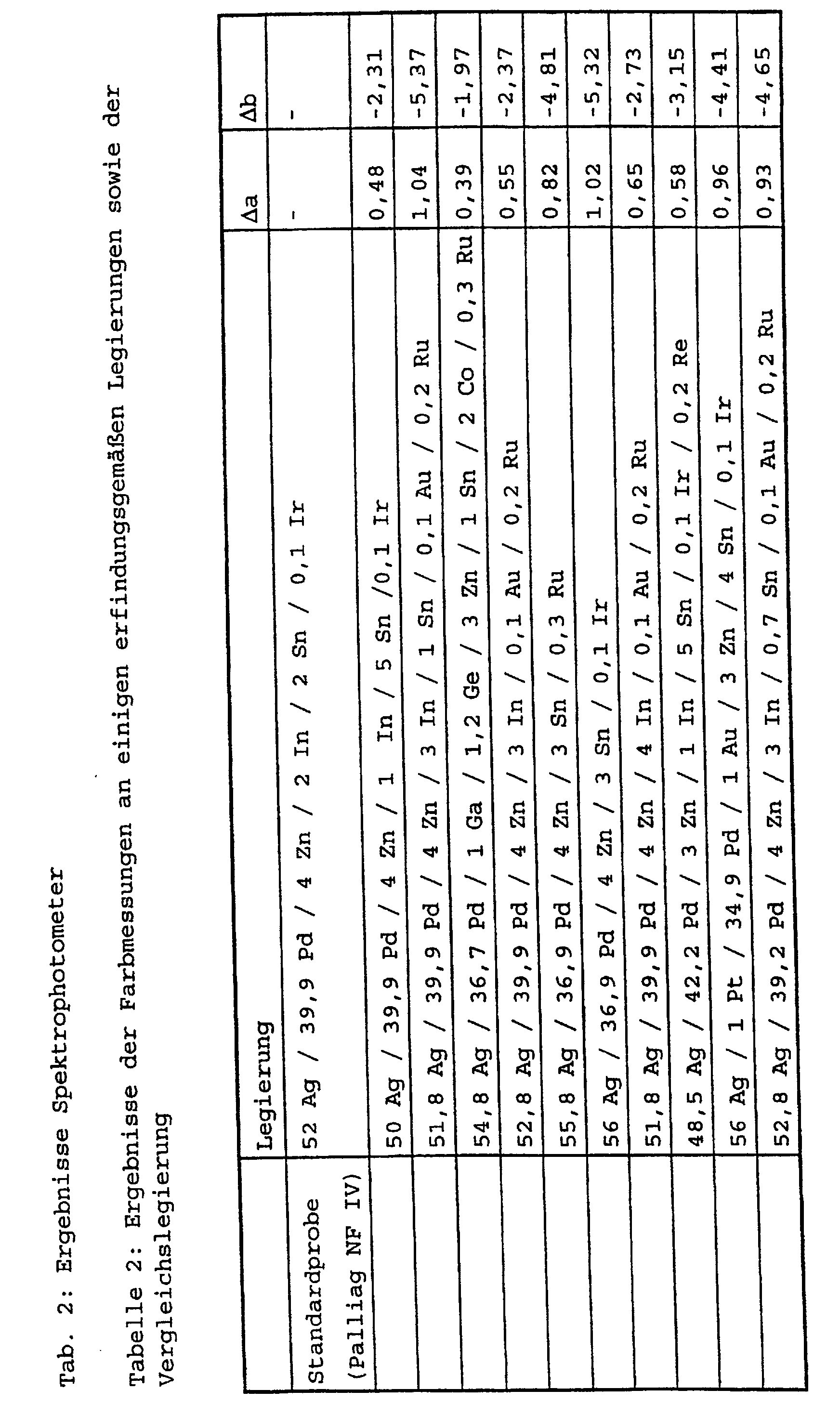

## Patentansprüche

1. Verwendung von Silber-Palladium-Legierungen, bestehend aus 45-60 Gew.% Ag, 30-45 Gew.% Pd, 0-5 Gew.% Au, 0-5 Gew.% Pt, 0-3 Gew.% Ge, 0-3 Gew.% Cu, 0-7 Gew.% Ga, 0-5 Gew.% Co, 0-1 Gew.% Mo, 0-1 Gew.% Ir, 0-1 Gew.% Ru, 0-1 Gew.% Re und je 0-6 Gew.% In, Sn und Zn, wobei sich alle Bestandteile zu 100 % ergänzen, die **dadurch gekennzeichnet sind, daß** entweder bei einem Gehalt von 0-1 Gew.% In gleichzeitig der Gehalt an Sn 1-6 Gew.% und der Gehalt an Zn 2-6 Gew.% oder bei einem Gehalt von 3-6 Gew.% In gleichzeitig der Gehalt an Sn 0-4 Gew.% und der Gehalt an Zn 4-6 Gew.% beträgt, zur Herstellung von mit niedrigschmelzender Dentalkeramik mit einem Wärmeausdehnungskoeffizient von ca. 16,5 µm/mK verblendbarem Zahnersatz.

2. Verwendung nach Anspruch 1 von Silber-Palladium-Legierungen,
**dadurch gekennzeichnet,**
**daß** sie 50-60 Gew.% Ag, 32-45 Gew.% Pd, 0-1 Gew.% In, 3-5 Gew.% Sn und 3-5 Gew.% Zn enthalten.

3. Verwendung nach Anspruch 2 von Silber-Palladium-Legierungen,
**dadurch gekennzeichnet,**
**daß** sie 55-57 Gew.% Ag, 35-38 Gew.% Pd, 3-5 Gew.% Sn und 3-5 Gew.% Zn enthalten.

4. Verwendung nach Anspruch 1 von Silber-Palladium-Legierungen
**dadurch gekennzeichnet,**
**daß** sie 50-60 Gew.% Ag, 32-45 Gew.% Pd, 3-6 Gew.% In, 0-2 Gew.% Sn und 4-6 Gew.% Zn enthalten.

5. Verwendung nach Anspruch 4 von Silber-Palladium-Legierungen
**dadurch gekennzeichnet,**
**daß** sie 50-55 Gew.% Ag, 38-42 Gew.% Pd, 3-5 Gew.% In, 0,5-2 Gew.% Sn und 4-5 Gew.% Zn enthalten.

6. Verwendung von Silber-Palladium-Legierungen gemäß den Ansprüchen 1 bis 5 und niedrigschmelzender Dentalkeramik mit einem Wärmeausdehnungskoeffizient von ca. 16,5 µm/mK zur Herstellung von mit Dentalkeramik verblendetem, verfärbungsfreien Zahnersatz.

7. Festsitzender und herausnehmbarer Zahnersatz,
**dadurch gekennzeichnet,**
**daß** eine Substruktur aus einer Silber-Palladium-Legierung gemäß den Ansprüchen 1 bis 5 mit einer niedrigschmelzenden Dentalkeramik mit Wärmeausdehnungskoeffizient von ca. 16,5 µm/mK verblendet ist.

## Claims

1. Use of silver-palladium alloys, consisting of 45-60 wt.% of Ag, 30-45 wt.% of Pd, 0-5 wt.% of Au, 0-5 wt.% of Pt, 0-3 wt.% of Ge, 0-3 wt.% of Cu, 0-7 wt.% of Ga, 0-5 wt.% of Co, 0-1 wt.% of Mo, 0-1 wt.% of Ir, 0-1 wt.% of Ru, 0-1 wt.% of Re and 0-6 wt.% in each case of In, Sn and Zn, wherein all constituents add up to 100%, which are **characterized in that** either with an In content of 0-1 wt.% the Sn content is 1-6 wt.% and the Zn content 2-6 wt.% at the same time or with an In content of 3-6 wt.% the Sn content is 0-4 wt.% and the Zn content 4-6 wt.% at the same time, for the manufacture of dental prostheses which can be faced with low-melting dental ceramic with a coefficient of thermal expansion of approx. 16.5 µm/mK.

2. Use according to Claim 1 of silver-palladium alloys,
**characterized in**
**that** they contain 50-60 wt.% of Ag, 32-45 wt.% of Pd, 0-1 wt.% of In, 3-5 wt.% of Sn and 3-5 wt.% of Zn.

3. Use according to Claim 2 of silver-palladium alloys,
**characterized in**
**that** they contain 55-57 wt.% of Ag, 35-38 wt.% of Pd, 3-5 wt.% of Sn and 3-5 wt.% of Zn.

4. Use according to Claim 1 of silver-palladium alloys,
**characterized in**
**that** they contain 50-60 wt.% of Ag, 32-45 wt.% of Pd, 3-6 wt.% of In, 0-2 wt.% of Sn and 4-6 wt.% of Zn.

5. Use according to Claim 4 of silver-palladium alloys,
**characterized in**
**that** they contain 50-55 wt.% of Ag, 38-42 wt.% of Pd, 3-5 wt.% of In, 0.5-2 wt.% of Sn and 4-5 wt.% of Zn.

6. Use of silver-palladium alloys according to Claims 1 to 5 and low-melting dental ceramic with a coefficient of thermal expansion of approx. 16.5 µm/mK for the manufacture of discoloration-free dental prostheses faced with dental ceramic.

7. Fixed and removable dental prostheses,
**characterized in**
**that** a substructure comprising a silver-palladium alloy according to the Claims 1 to 5 is faced with a low-melting dental ceramic with a coefficient of thermal expansion of approx. 16.5 µm/mK.

## Revendications

1. Utilisation d'alliages argent-palladium, constitués de 45 - 60 % en poids de Ag, 30 - 45 % en poids de Pd, 0 - 5 % en poids de Au, 0 - 5 % en poids de Pt, 0 - 3 % en poids de Ge, 0-3 % en poids de Cu, 0 - 7 % en poids de Ga, 0 - 5 % en poids de Co, 0 - 1 % en poids de Mo, 0 - 1 % en poids de Ir, 0 - 1 % en poids de Ru, 0 - 1 % en poids de Re, et dans chaque cas 0-6 % en poids de In, Sn et Zn, tous les constituants se complétant à 100 %,
**caractérisés en ce que**
pour une teneur de 0 - 1 % en poids de In, en même temps la teneur en Sn est de 1 - 6 % en poids et la teneur en Zn de 2 - 6 % en poids ou pour une teneur de 3 - 6 % en poids de In, en même temps la teneur en Sn est de 0 - 4 % en poids et la teneur en Zn est de 4 - 6 % en poids pour la fabrication d'une prothèse dentaire pouvant être revêtue avec une céramique dentaire à bas point de fusion présentant un coefficient de dilatation thermique d'environ 16,5 µm/mK.

2. Utilisation selon la revendication 1 d'alliages argent-palladium,
**caractérisés en ce qu'**
ils contiennent 50 - 60 % en poids de Ag, 32 - 45 % en poids de Pd, 0 - 1 % en poids de In, 3-5 % en poids de Sn et 3- 5 % en poids de Zn.

3. Utilisation selon la revendication 2 d'alliages argent-palladium,
**caractérisés en ce qu'**
ils contiennent 55 - 57 % en poids de Ag, 35 - 38 % en poids de Pd, 3 - 5 % en poids de Sn et 3 - 5 % en poids de Zn.

4. Utilisation selon la revendication 1, d'alliages argent-palladium,
**caractérisés en ce qu'**
ils contiennent 50 - 60 % en poids de Ag, 32 - 45 % en poids de Pd, 3 - 6 % en poids de In, 0-2 % en poids de Sn et 4 - 6 % en poids de Zn.

5. Utilisation selon la revendication 1 d'alliages argent-palladium,
**caractérisés en ce qu'**
ils contiennent 50 - 55 % en poids de Ag, 38 - 42 % en poids de Pd, 3 - 5 % en poids de In, 0,5 - 2 % en poids de Sn, et 4 - 5 % en poids de Zn.

6. Utilisation d'alliages argent-palladium selon les revendications 1 à 5 et de céramique dentaire à bas point de fusion présentant un coefficient de dilatation thermique d'environ 16,5 µm/mK pour la fabrication d'une prothèse dentaire revêtue de céramique dentaire ne changeant pas de couleur.

7. Prothèse dentaire fixe et amovible,
**caractérisée en ce qu'**
une structure constituée d'un alliage argent-palladium selon les revendications 1 à 5 est revêtue d'une céramique dentaire à bas point de fusion, présentant un coefficient de dilatation thermique d'environ 16,5 µm/mK.
